# EUROPEAN PATENT SPECIFICATION

(11) **EP 1 542 747 B1**
(45) Date of publication and mention of the grant of the patent: **29.11.2006**
(21) Application number: 03754789.0
(22) Date of filing: 22.09.2003
(51) Int. Cl.: A61M 5/50

(54) **Single use syringe with plunger rod locking mechanism**
Einwegspritze mit Kolbenstangeverriegelungsvorrichtung
Seringue à usage unique avec dispositif de verrouillage de la tige de piston

(30) Priority: 25.09.2002 US 254924
(43) Date of publication of application: 22.06.2005
(73) Proprietor: Becton Dickinson and Company, Franklin Lakes, New Jersey 07417-1880 (US)
(72) Inventor: PELKEY, Brian J., Rockaway, NJ 07866 (US); FLEISCHER, Gene, New City, NY 10956 (US)
(74) Representative: Weber, Thomas
(86) International application number: PCT/US2003/029626
(87) International publication number: WO 2004/028604

(56) References cited:
- EP-A- 0 308 040
- US-A- 4 961 728
- US-A- 5 989 219

## Description

### BACKGROUND OF THE INVENTION

### Field of the Invention

The field of the invention relates to single use syringes and locking devices for locking the plunger assemblies of such syringes.

### Brief Description of the Related Art

In the United States and throughout the world the multiple use of hypodermic syringe products that are intended for single use only is instrumental in drug abuse and more particularly in the transfer of diseases. Intravenous drug users who routinely share and reuse syringes are a high risk group with respect to the AIDS virus. Also, the effects of multiple use are a major concern in under-developed countries where repeated use of syringe products may be responsible for the spread of many diseases. Reuse of single use hypodermic syringe assemblies is also instrumental in the spread of drug abuse even in the absence of infection or disease.

Many attempts have been made to remedy this problem. Some of these attempts have required a specific act to destroy the syringe after use either by using a destructive device or providing a syringe assembly with frangible zones so that the syringe could be rendered inoperable by the application of force. Other attempts have involved the inclusion of structure which would allow the destruction or defeating of the syringe function through a conscious act by the syringe user. Although many of these devices work quite well, they do require the specific intent of the user followed by the actual act to destroy or render the syringe inoperable. None of these devices is effective with a user having the specific intent to reuse the hypodermic syringe.

Single use hypodermic syringes that become inoperative or incapable of further use automatically without any additional act on the part of the user have been developed. One such syringe is disclosed in U.S. Patent No. 4,961,728. The syringe disclosed in this patent includes a locking element positioned in the syringe barrel. The locking element includes proximally and outwardly facing barbs that engage the inner surface of the syringe barrel and an inwardly facing driving edge adapted to interact with the plunger rod to move the locking element along the barrel as the stopper is advanced. The plunger rod includes a ledge positioned at a distance from the proximal side of a support wall that approximates the length of the locking element. The driving edge of the locking element engages the ledge, thereby ensuring that the locking element moves distally with the plunger rod and stopper. A syringe including a similar locking element is disclosed in U.S. Patent No. 5,989,219.

U.S. Patent Nos. 5,021,047, 5,062,833 and 5,562,623 disclose single use syringes having plunger rods that have teeth or ridges and locking elements that engage the teeth or ridges. The locking elements of these syringes also include outwardly extending teeth or prongs that engage the inside surface of the syringe barrel. The plunger rods of these syringes can be retracted to draw fluid into the syringe barrel while the locking elements remain stationary. Distal movement of the plunger rods causes the fluid to be expelled, the locking elements moving distally with the plunger rods and substantially preventing further plunger rod retraction.

Syringes are available in many different sizes, and have plunger rods that are commensurate in size with the syringe barrels in which they are used. This generally necessitates the use of a different size locking element for each size syringe.

### SUMMARY OF THE INVENTION

The invention relates to a syringe assembly including a locking element capable of locking a plunger rod with respect to a syringe barrel. The assembly includes a syringe barrel, a plunger rod assembly and a locking element. The syringe barrel includes an inside surface defining a chamber, an open end, and a distal end. The plunger rod assembly includes an elongate body portion and a stopper. The locking element is slidably positioned within the chamber of the syringe barrel, engaging the inside surface thereof such that the locking element is substantially immovable in the direction of the open end of the syringe. It is also engageable with the plunger rod assembly such that the plunger rod assembly and locking element can be moved distally together toward the distal end of the syringe barrel. In a preferred embodiment, the plunger rod assembly can initially be moved proximally with respect to the locking element to aspirate fluid into the syringe barrel. The body portion of the plunger rod includes one or more axially extending recesses. Each recess is defined by a pair of converging surfaces. At least one of the recesses is defined by surfaces that converge along a line that is displaced from the longitudinal axis of the plunger rod. The locking element is positioned within the recess and is engageable with one or both converging surfaces. It accordingly is movable in the distal direction with the plunger assembly.

### BRIEF DESCRIPTION OF THE DRAWINGS

Fig. 1 is an exploded, perspective view of a single use syringe assembly according to the invention;

Fig. 2 is a top perspective view showing the syringe assembly as provided to a user;

Fig. 3 is a top perspective view thereof showing the syringe assembly following retraction of the plunger rod assembly thereof;

Fig. 4 is a top perspective view of the syringe assembly with the plunger rod assembly in a locked position following an injection;

Fig. 5 is a top perspective view thereof showing the operation of a tamper-resistance feature of the syringe assembly upon attempted retraction of the plunger rod assembly from the locked position of Fig. 4;

Fig. 6 is a partial cross-sectional view thereof showing the plunger rod assembly in a position prior to initial retraction;

Fig. 6A is an enlarged cross-sectional view of the distal end of the syringe assembly of Fig. 6;

Fig. 7 is a partial cross-sectional view thereof showing the plunger rod assembly as retracted by the user to draw fluid into the syringe barrel;

Fig. 7A is an enlarged cross-sectional view showing the distal end of the plunger rod assembly;

Fig. 8 is a partial cross-sectional view of the syringe assembly following the injection stroke;

Fig. 8A is an enlarged cross-sectional view of the distal end thereof;

Fig. 9 is an enlarged cross-sectional view thereof showing the operation of a tamper-resistance feature upon attempted retraction of the plunger rod assembly from the position shown in Fig. 8;

Fig. 10 is a cross-sectional view taken along line 10-10 of Fig. 6;

Fig. 11 is a perspective view of a plunger rod assembly in accordance with a preferred embodiment of the invention;

Fig. 12 is a perspective view of a locking element;

Fig. 13 is another perspective view of the locking element;

Fig. 14 is an enlarged elevation view of the distal end of the locking element;

Fig. 15 is a side elevation view of the locking element;

Fig. 16 is a cross-sectional view of the locking element taken along line 16-16 of Fig. 15;

Fig. 17 is a cross-sectional view of the locking element taken along line 17-17 of Fig. 15; and

Fig. 18 is an end view of the locking element.

### DETAILED DESCRIPTION OF THE INVENTION

There is shown in the drawings and will be described in detail herein a preferred embodiment of the invention with the understanding that the present disclosure is to be considered as exemplary of the principles of the invention and is not intended to limit the invention to the embodiment illustrated.

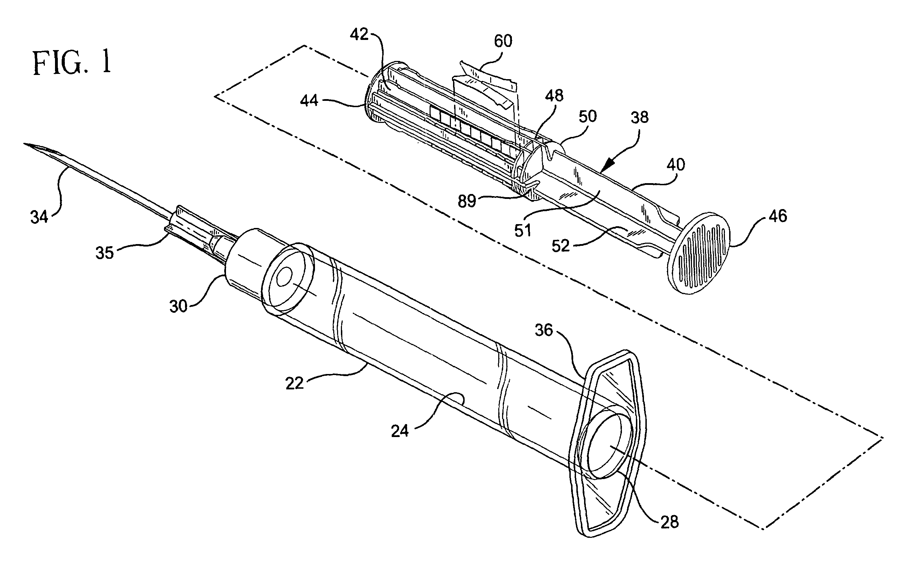

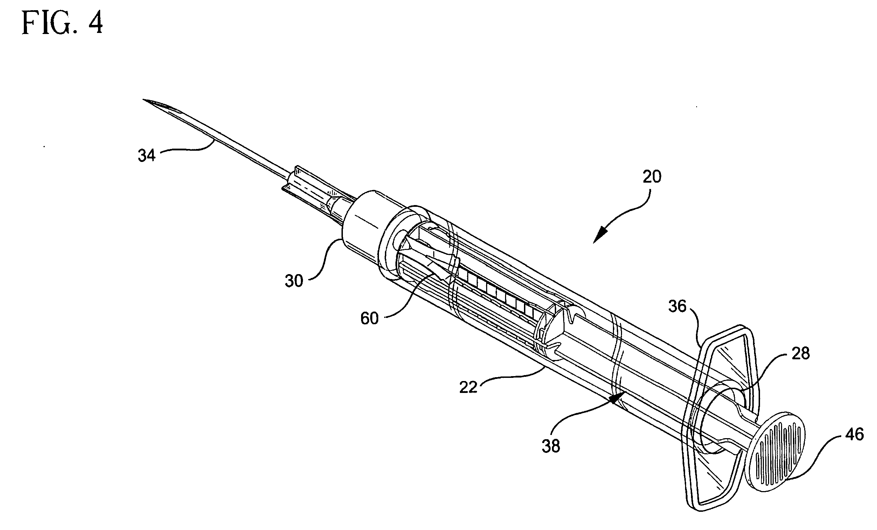

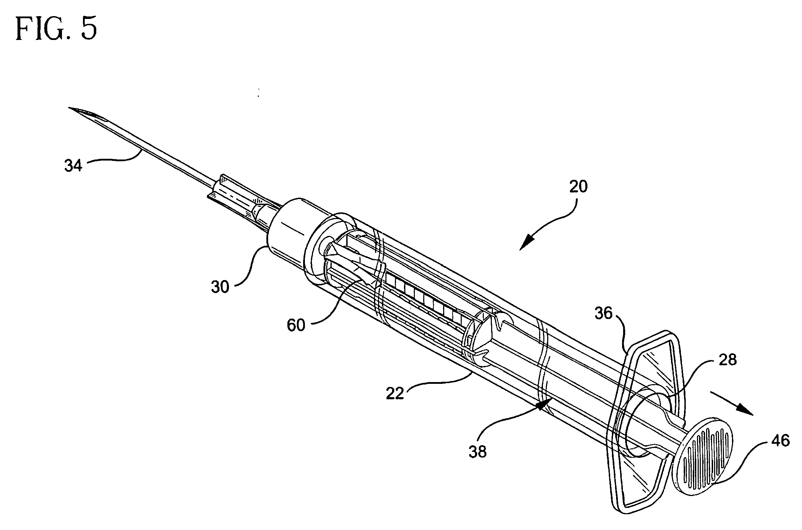

Referring first to Figs. 1-9, a single use syringe assembly 20 includes a barrel 22 having an inside surface 24 defining a chamber 26 for retaining fluid. The barrel 22 includes an open end 28 and a distal end 30 having a passageway 32 therethrough in communication with the chamber. A needle cannula 34 projects outwardly from the distal barrel end. The needle cannula has a lumen (not shown) therethrough in fluid communication with the passageway and a sharpened distal tip. The syringe assembly of the present invention is shown with a needle cannula assembly including a hub 35 that.is removably attached to the distal end of the barrel. It will be appreciated that the invention could be applied to syringe assemblies having permanently affixed needles or needle/hub assemblies, or fixed or removable blunt cannulas. While intended for reconstitution of vaccines, the syringe assembly can be used for general curative purposes or other purposes.

As used in the preceding paragraph and hereafter, the term "distal end" refers to the end furthest from the person holding the syringe assembly. The term "proximal end" refers to the end closest to the holder of the syringe assembly. In the preferred embodiment, the proximal end of the barrel 22 includes a flange 36 to facilitate handling and positioning of the syringe assembly and to maintain the relative position of the barrel with respect to the plunger rod during filling and medication administration.

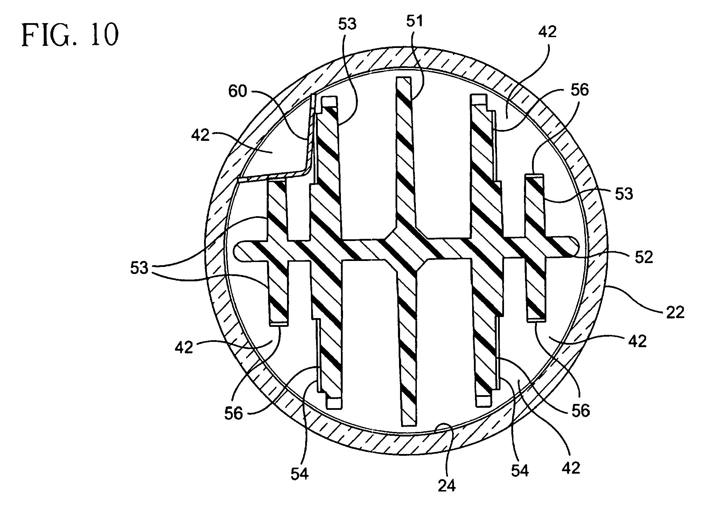

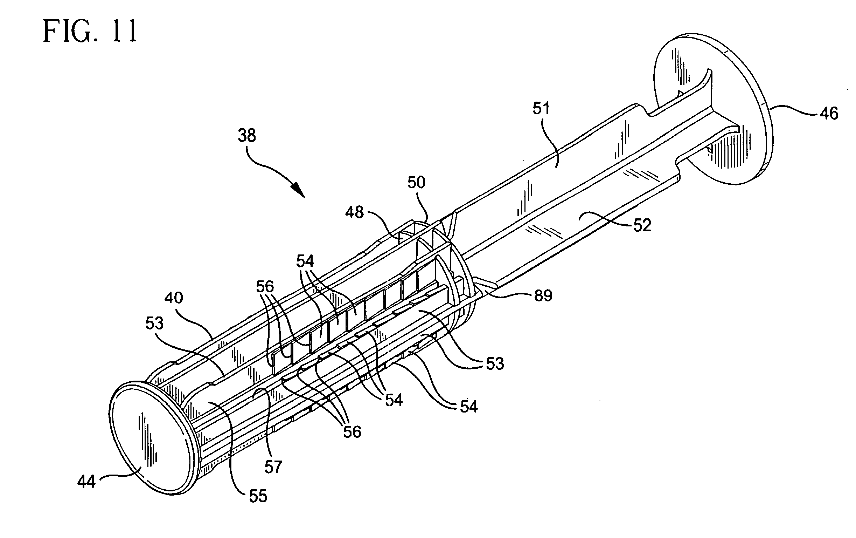

A plunger rod assembly 38 used in the syringe assembly 20 includes an elongate body portion 40 including a plurality of elongate recesses 42 running substantially parallel to the longitudinal axis of rotation thereof. The distal end of the elongate body portion includes an integral stopper 44. A disc-shaped flange 46 is provided at the proximal end of the plunger rod for allowing the user to apply the force necessary to move the plunger rod with respect to the barrel. The elongate body portion 40 includes a pair of discs 48, 50 intermediate the proximal and distal ends thereof. The sections between the relatively proximal disc 50 and the flange 46 and the relatively distal disc 48 and the stopper 44 include radially extending walls 51, 52, both of which preferably traverse the longitudinal axis of rotation of the plunger rod assembly 38. Additional walls 53 that resemble fins extend perpendicularly from both sides of one wall 52 of the radially extending walls in the section between the distal disc 48 and stopper 44. In this preferred embodiment, the walls 53 are substantially parallel to each other. The areas between walls could be filled in to provide additional rigidity if necessary. Ratchet-like teeth 54 are formed on selected surfaces of the walls 53, as best shown in Figs. 10 and 11. Each tooth 54 includes a distally facing surface or shoulder 56. The wall surfaces 55, 57 including the teeth converge along an imaginary line that runs substantially parallel to the longitudinal axis of the plunger rod assembly, but radially displaced therefrom. As shown in the drawings, the surfaces 55, 57 do not necessarily adjoin each other. They together define recesses 42 for positioning of a locking element 60. While four recesses are provided, a greater or lesser number may be employed. It will be appreciated that the recesses 42 can be formed by surfaces that actually meet along the line of convergence. It will further be appreciated that while the plunger rod assembly as shown and described herein is of integral construction, it may in fact be comprised of two or more separate elements. The stopper may, for example, be a separate component made from a material that is different from the material comprising the remainder of the plunger rod assembly. In this preferred embodiment, the syringe barrel is comprised of polypropylene, and contains an internal lubricant, and the plunger rod assembly is comprised of polyethylene.

The locking element 60 is positioned within the barrel 22 and within one of the elongate recesses 42 defined by the additional walls 53. The recess 42 acts as a pathway for longitudinal motion of the locking element relative to the plunger rod assembly. As the recesses are displaced from the longitudinal axis of the plunger rod assembly, the same size locking element can be used that is employed in a smaller syringe. In the smaller syringe, the locking element would be positioned in a recess adjoining the longitudinal axis or at least closer to this axis than in the syringe disclosed herein. U.S. Patent Nos. 4,961,728 and 5,989,219 disclose the placement of a locking element at or near the longitudinal axis. A syringe as disclosed in these patents could be provided for administering doses of about 0.5ml. The invention allows the same size locking element to be used in such very small syringes as well as those exceeding five milliliters.

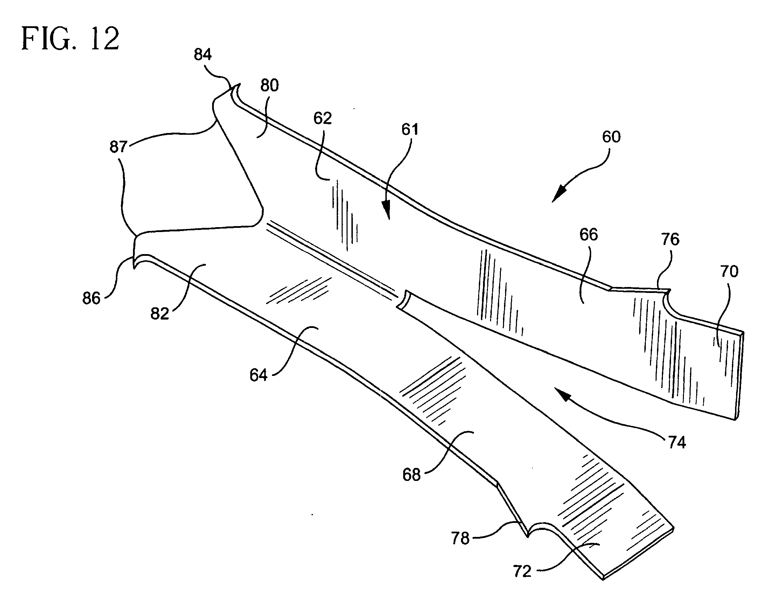

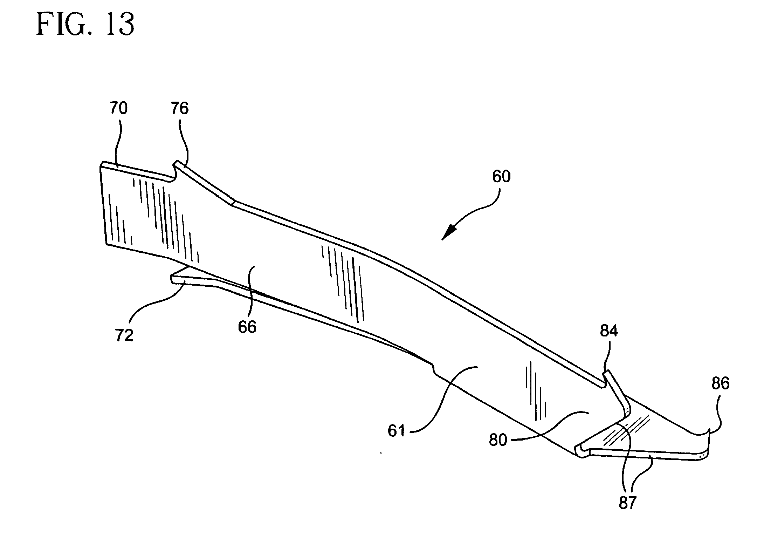

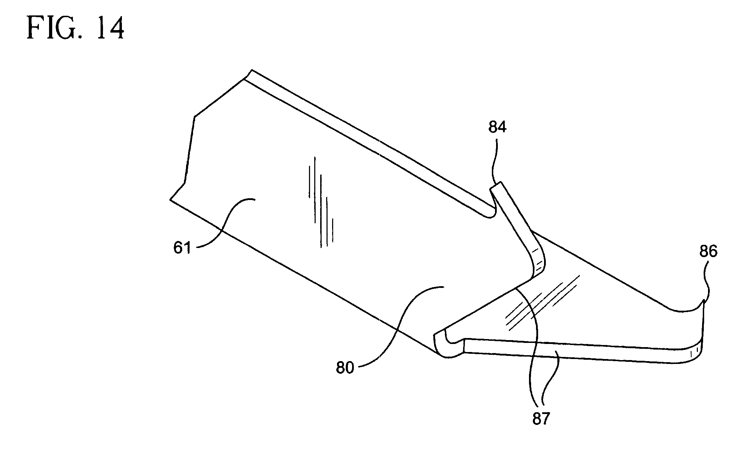

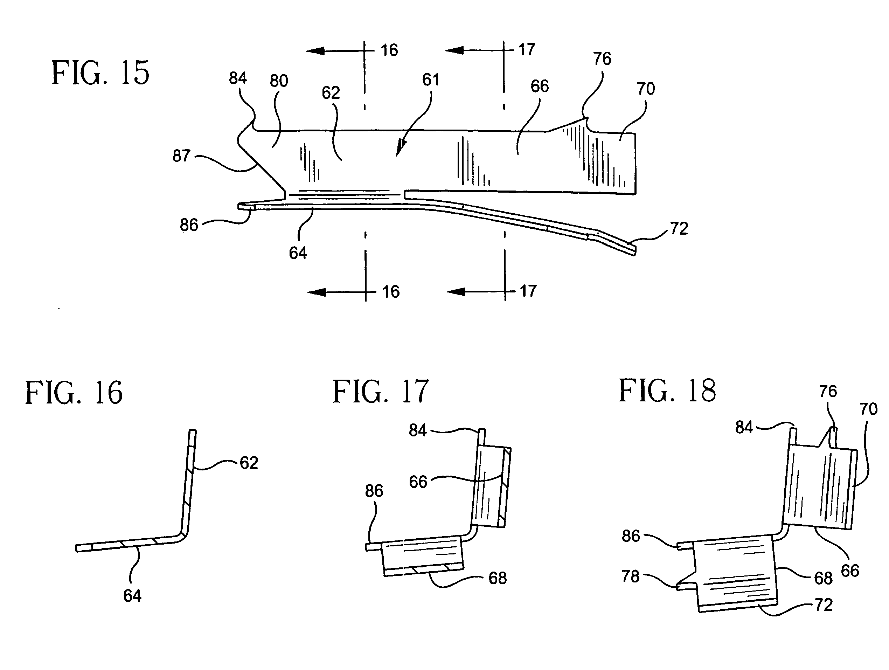

The locking element 60, as best shown in Figs. 12-18, includes a generally V-shaped body portion 61 comprising first and second radially extending walls 62, 64 joined along a longitudinal axis. The walls 62, 64 preferably form an angle of greater than ninety degrees, and preferably about one hundred degrees. A first leg 66 extends proximally from the first wall and a second leg 68 extends proximally from the second wall 64. The legs flare outwardly with respect to the V-shaped body portion 61, as best shown in Fig. 15. The legs 66, 68 are preferably substantially longer than the length of the body portion 61. In a locking element having an overall length of about seventeen millimeters, the legs 66, 68 may be about ten millimeters in length.

Each of the legs 66, 68 includes a proximal end portion 70, 72 that is angled towards one of the walls 53 of the plunger rod assembly. They further include inner and outer edges. (The terms "inner" and "outer" are relative terms as used herein.) The inner edges thereof are substantially adjacent to each other, separated by a longitudinal gap 74. Barbs 76, 78 are integral with the outer edges of the first and second legs. The barbs face proximally, and are preferably located slightly distally of the angled end portions 70, 72. The barbs may be different in appearance from those shown in the drawings so long as they are capable of engaging the inside surface 24 of the syringe barrel to prevent proximal movement of the locking element.

A second pair of legs extends distally from the V-shaped body portion 61. One of these legs 80 extends from the first wall 62 and the other 82 from the second wall 64. Barbs 84, 86 extend proximally from the distal ends of the legs 80, 82. The barbs are formed on the outer edges of the distally extending legs. Each leg further includes a cutting edge 87 capable of penetrating the stopper 44.

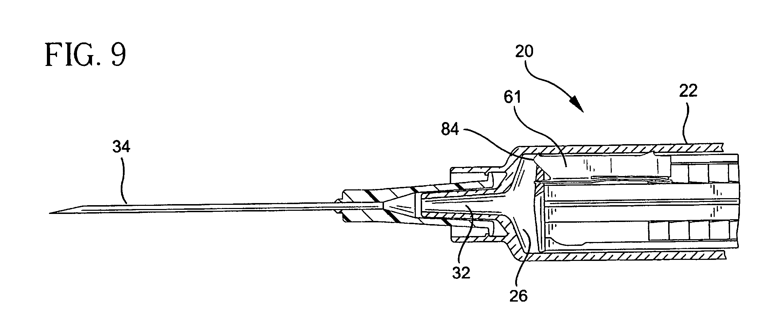

The locking element is preferably formed from a thin sheet of metal such as stainless steel. The thickness in a preferred embodiment thereof is about 0.20 mm. The dimensions of the locking element are selected in accordance with the barrels and plunger rod assemblies with which it is to be used. The angle formed between the two halves of the locking element, as shown in Fig. 16, is preferably greater than ninety degrees, and preferably about one hundred degrees. When placed in one of the recesses 42 in the plunger rod assembly, the locking element will accordingly exert a force against the two of the converging wall surfaces 55, 57 that define the recess. The cutting edges 87 are preferably formed by providing bevels on one side of the substrate from which the locking element is constructed. It will be appreciated that the substrate could be ground on both sides thereof to form cutting edges for disabling the stopper 44. Alternatively, barbs (not shown) or other cutting members can be provided on the locking element for piercing the stopper. Fig. 9 shows the penetration of the stopper by the cutting edges that will occur should one attempt to reuse the syringe following an injection.

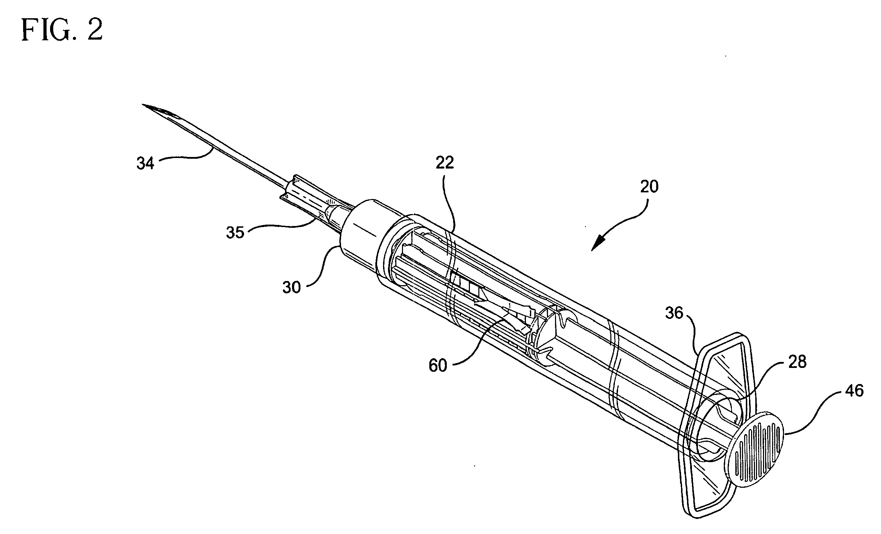

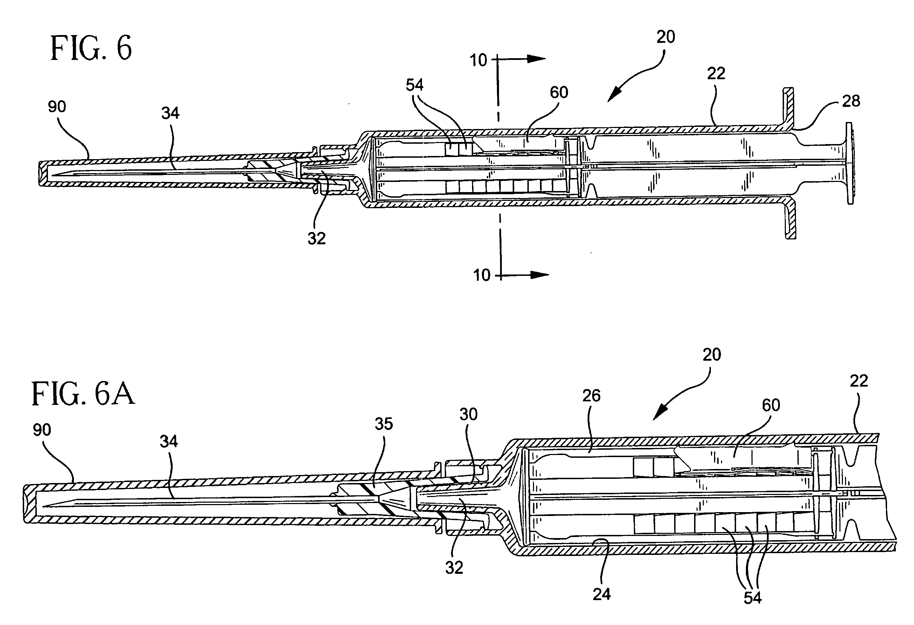

The syringe assembly is easily constructed from the component parts thereof. The locking element 60 is positioned in one of recesses 42 in the plunger rod assembly such that the angled end portions of the legs 66, 68 adjoin the relatively distal disk 48, as shown in Figs. 2 and 6. Alternatively, the end portions of the legs can initially abut selected teeth 54 should a smaller dosage be required. The legs 66, 68 and spring member extend proximally, and the barbs 76, 78, 84, 86 are angled proximally with respect to the plunger rod assembly. The plunger rod/locking element assembly is then inserted into the barrel 22 through the proximal end thereof. As the assembly is moved distally within the barrel, the angular orientation of the barbs allows them to slide along while engaging inside surface 24 of the barrel. The locking element moves distally with the plunger rod due to the engagement of the ends of legs 66, 68 with the disc 48. The gap 74 is maintained between the legs 66, 68 even after installation of the locking element. The maintenance of the gap and the relatively long lengths of the legs, which act as cantilever springs, provide a relatively reduced force on the barrel and facilitate use and installation. The plunger rod/locking element assembly is moved distally until the stopper engages the end wall of the barrel as shown in Figs. 2 and 6. It is then ready for use or storage. A needle cover 90 can be mounted to the distal end of the barrel to protect the needle cannula. The cover is removed prior to use.

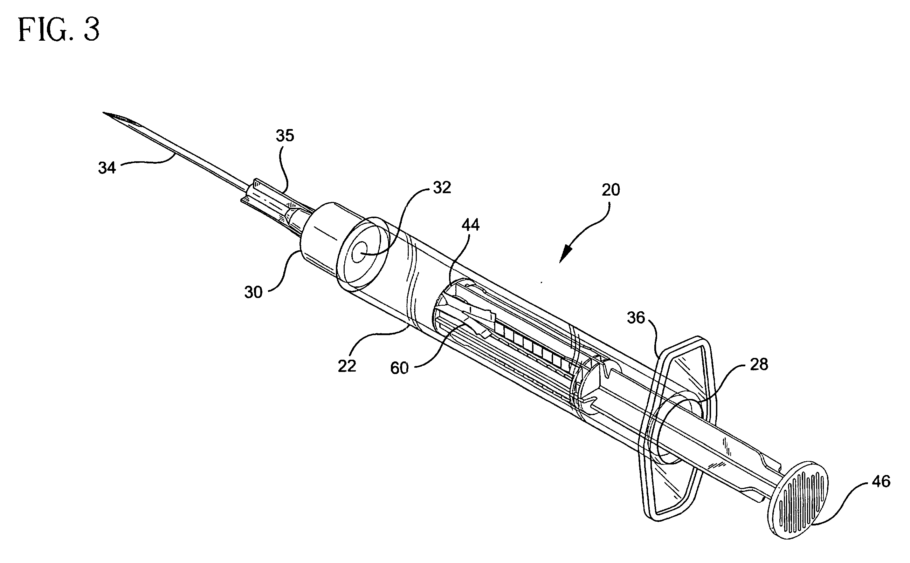

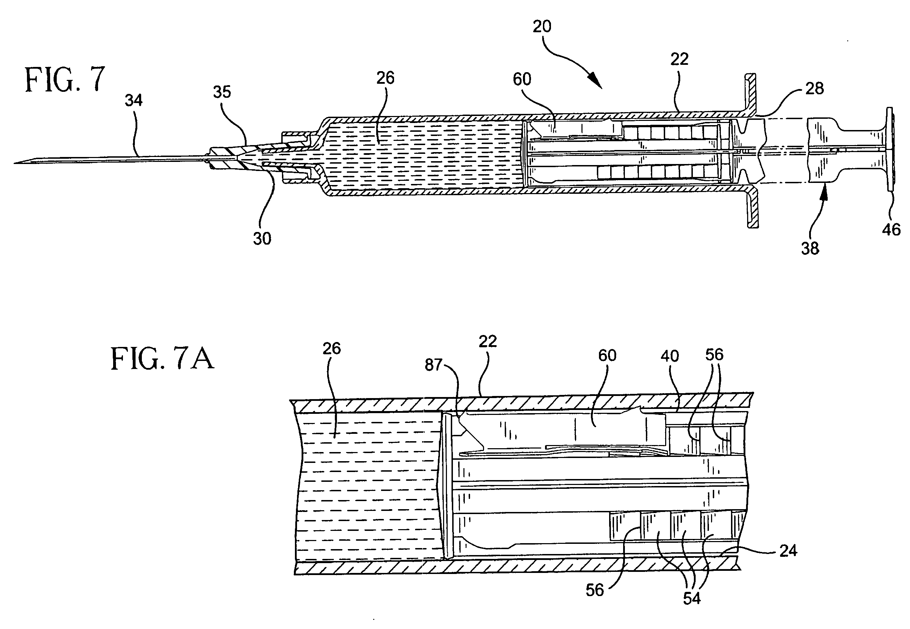

In use, plunger rod assembly 38 is retracted from the position shown in Figs. 2 and 6 to the position shown in Figs. 3 and 7 in order to draw fluid through needle cannula 34 and passageway 32 and into chamber 26 of barrel 22. Locking element 60 remains stationary during such retraction, and the plunger rod assembly is moved proximally with respect to both barrel 22 and the locking element. This is due to the engagement of barbs 76, 78, 84, 86 with inside surface 24 of the barrel. The barbs are preferably made from a harder material than the barrel, which enhances their ability to resist proximal movement. Angled ends 70, 72 of legs 66, 68 of the locking element ride over teeth 54 of the plunger rod assembly during retraction thereof. The user may feel and/or hear the movement of the legs over the teeth.

Retraction of the plunger rod assembly 38 is limited by the locking element. As shown in Figs. 7 and 7A, the proximal surface of stopper 44 engages the distal end of locking element 60. The user can feel this engagement. Cutting edges 87 do not penetrate the stopper as a result of the forces exerted during normal use. As the locking element cannot be moved proximally, further retraction of the plunger rod assembly is resisted. The amount of fluid that can be drawn into chamber 26 is accordingly limited by the distance between the proximal surface of the stopper and the disc 48 as well as the length of the locking element. It will be appreciated that the distance between the stopper and the relatively distal disc 48 and the length of the locking element 54 can be chosen to meet the needs of particular applications.

The proximal end portions of legs 66, 68 of the locking element adjoin the end of a relatively distal tooth 54 when the plunger rod assembly is retracted to the position shown in Figs. 7 and 7A. The distance between this end of the tooth 54 and the distal end surface of the relatively distal disc 48, being substantially the same as the distance between the distal end of the locking element and the proximal end portions of the legs, causes the locking element to be substantially immovable with respect to the plunger rod assembly. As discussed above, the locking element is substantially immovable in the proximal direction within the barrel due to the engagement of the barbs with the inside surface of the barrel 22. The syringe can also be provided to the end user as a prefilled syringe, in which case retraction of the plunger rod assembly would not be possible.

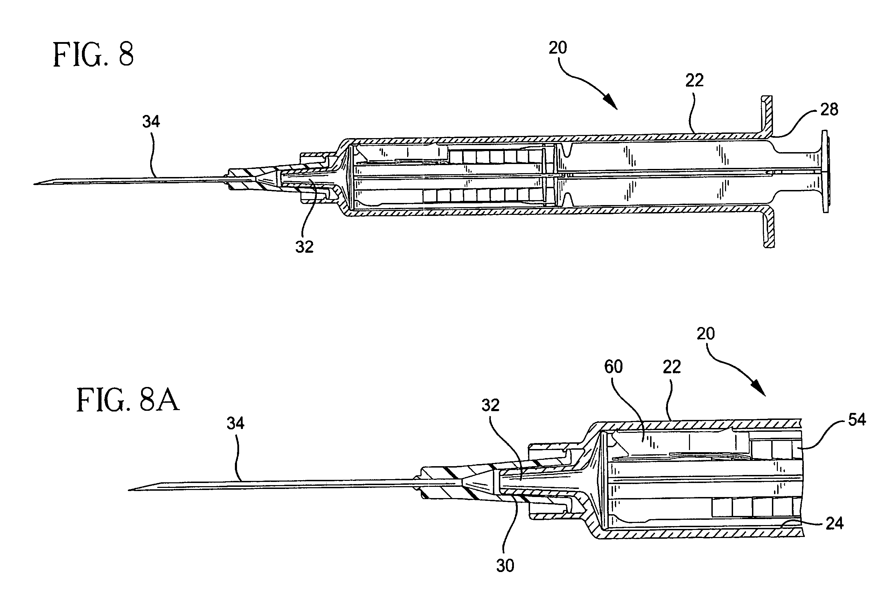

Once the fluid has been drawn into the barrel from a vial or other fluid source, the needle cannula can be removed from the fluid source and used for injection. During the injection of a patient, plunger assembly 38 and locking element both move distally from the positions shown in Figs. 7 and 7A to the positions shown in Figs. 8 and 8A. In Figs. 8 and 8A, stopper 44 again adjoins or engages the end wall of barrel 22. Locking element 54 remains positioned between disc 48 and most distal ratchet tooth 54. Both plunger rod assembly 38 and the locking element are substantially immovable from their positions. Syringe assembly 20 accordingly cannot be reused. Should a person use extraordinary force in an attempt to retract the plunger rod assembly from the position shown in Figs. 8 and 8A, the cutting edges 87 at the distal end of the locking element will penetrate the stopper, rendering it unusable, as shown in Fig. 9. Disabling of the stopper preferably occurs when the force exerted is approximately sufficient to dislodge the locking element in the proximal direction, or a lesser force. As discussed above, simple engagement of the cutting edges and stopper should not compromise the integrity of the stopper.

An additional tamper-resistance feature is comprised of notches 89 in the plunger rod assembly. If the plunger rod assembly is twisted forcefully, it will break prior to disablement of locking element 60.

The syringe barrel of the present invention may be constructed of a wide variety of rigid materials with thermoplastic materials such as polypropylene and polyethylene being preferred. Similarly, thermoplastic materials such as polypropylene, polyethylene and polystyrene are preferred for the plunger rod and integral stopper. A wide variety of materials such as natural rubber, synthetic rubber, thermoplastic elastomers and combinations thereof are suitable for the stopper if the stopper is manufactured as a separate component. The choice of stopper material will depend on compatibility with the medication being used.

As previously recited, it is preferable that the locking element be fabricated from a material which is harder than the barrel so that the locking barbs may effectively engage the barrel. Resilient spring like properties are also desirable along with low cost, dimensionally consistent fabrication. With this in mind, sheet metal is the preferred material for the locking element with stainless steel being preferred for medical applications. Although the locking element of the preferred embodiment is fabricated from a single sheet, it is within the purview of the instant invention to include locking elements made of other forms and/or containing multiple parts. Locking elements having structures other than that shown and described herein could also be successfully employed. One such locking element is disclosed in U.S. Patent No. 5,989,219. The distal end of the locking element disclosed in the patent could be provided with a cutting edge similar to those described above. Alternatively, barbs (not shown) could be provided at the distal end of the locking element for rendering the stopper unusable.

The syringe barrel employed in accordance with the invention may have a varying wall thickness along its length. The portion of the barrel used for containing medication could be relatively thin and resilient to ensure proper sealing with the stopper. The remainder of the barrel could be relatively thick and less resilient such that it would tend to crack if squeezed by pliers or another device used for attempted tampering. Sufficient barrel crystallinity is desirable in the area of the locking element to cause this area to crack upon deformation of the syringe barrel to an extent that would permit retraction of the plunger rod assembly with the locking element.

Thus, it can be seen that the present invention provides a simple, reliable, easily fabricated, single use syringe which becomes inoperable or incapable of further use without any additional act on the part of the user. It further allows the use of a locking element of the same size that is used on smaller or larger syringes.

## Claims

1. A syringe assembly comprising:
a syringe barrel having an inside surface defining a chamber, an open end, and a distal end;
a plunger rod assembly extending within said syringe barrel and including an elongate body portion having a longitudinal axis, a stopper connected to said elongate body portion, and an axially extending recess, said recess being bounded by a pair of surfaces and being radially displaced from the longitudinal axis of said elongate body portion, and
a locking element slidably positioned within said chamber and extending within said recess, said locking element engaging said inside surface of said syringe barrel such that said locking element is substantially immovable in the direction of the open end of said syringe barrel, said locking element further being engageable with at least one of said surfaces of said plunger rod assembly such that said plunger rod assembly and locking element can be moved distally together toward the distal end of said syringe barrel with said locking element being maintained in said recess and substantially displaced from the longitudinal axis of said plunger rod assembly.

2. The syringe assembly of claim 1 wherein said locking element is comprised of an integral, resilient metal structure, said locking element being positioned such that said plunger rod assembly can be moved proximally with respect to said locking element.

3. The syringe assembly of claim 1 wherein said locking element includes one or more proximally extending barbs engaging said inside surface of said syringe barrel, and said locking element and stopper are positioned such that said plunger rod assembly can be moved proximally with respect to said locking element.

4. The syringe assembly of claim 3 wherein said locking element includes a body portion having a distal end and proximal end, said body portion of said locking element being generally V-shaped and engageable with each of said pair of surfaces bounding said recess.

5. The syringe assembly of claim 4 including a first pair of legs extending from and deflectable with respect to said proximal end of said body portion, said legs engaging said plunger rod assembly.

6. The syringe assembly of claim 5 including a first proximally extending barb adjacent said distal end of said body portion and a second proximally extending barb extending from at least one of said legs, said first and second barbs engaging said inside surface of said syringe barrel.

7. The syringe assembly of claim 1 wherein said elongate body portion of said plunger rod assembly includes a plurality of radially extending walls that converge near said longitudinal axis and a first additional wall extending from one of said radially extending walls in a non-radial direction, said first additional wall defining one of said surfaces bounding said recess.

8. The syringe assembly of claim 7 including a second additional wall extending from one of said radially extending walls, said second additional wall defining one of said surfaces bounding said recess.

9. The syringe assembly of claim 8 wherein said second additional wall is substantially parallel to said first additional wall.

10. The syringe assembly of claim 1 wherein said elongate body portion of said plunger rod assembly includes a first wall proximal to said stopper and a plurality of second walls projecting from a first side of first wall, two of said second walls defining said surfaces of said recess.

11. The syringe assembly of claim 10 wherein at least one of said surfaces of said recess includes a plurality of abutments for engaging said locking element.

12. The syringe assembly of claim 10 wherein said second walls extend substantially perpendicularly with respect to said first wall.

13. The syringe assembly of claim 10 wherein said second walls extend from two sides of said first wall and define a plurality of recesses, each of said recesses capable of receiving said locking element.

14. The syringe assembly of claim 10 wherein said locking element includes a generally V-shaped body portion engageable with each of said pair of surfaces bounding said recess.

15. The syringe assembly of claim 12 wherein said locking element includes a generally V-shaped body portion engageable with each of said pair of surfaces bounding said recess.

## Patentansprüche

1. Spritzenanordnung mit:
einem Spritzenzylinder mit einer eine Kammer begrenzenden Innenfläche, einem offenen Ende und einem distalen Ende;
einer Kolbenstangenanordnung, die sich in dem Spritzenzylinder erstreckt und einen langgestreckten Körperteil mit einer Längsachse, einen mit dem langgestreckten Körperteil verbundenen Stopfen und eine axial verlaufende Ausnehmung aufweist, wobei die Ausnehmung von zwei Flächen begrenzt ist und gegenüber der Längsachse des langgestreckten Körperteils radial versetzt ist, und
einem Verriegelungselement, das gleitend verschiebbar in der Kammer angeordnet ist und sich in der Ausnehmung erstreckt, wobei das Verriegelungselement an der Innenfläche des Spritzenzylinders derart angreift, dass das Verriegelungselement im wesentlichen nicht in Richtung des offenen Endes des Spritzenzylinders bewegbar ist, wobei das Verriegelungselement ferner derart in Angriff an einer der Flächen der Kolbenstangenanordnung bringbar ist, dass die Kolbenstangeanordnung und das Verriegelungselement zusammen distal in Richtung des distalen Endes des Spritzenzylinders bewegbar sind, wobei das Verriegelungselement in der Ausnehmung gehalten und im wesentlichen gegenüber der Längsachse der Kolbenstangenanordnung versetzt ist.

2. Spritzenanordnung nach Anspruch 1, bei der das Verriegelungselement aus einer einstückigen elastischen Metallstruktur besteht, wobei das Verriegelungselement derart positioniert ist, dass die Kolbenstangenanordnung proximal in bezug auf das Verriegelungselement bewegbar ist.

3. Spritzenanordnung nach Anspruch 1, bei der das Verriegelungselement einen oder mehrere sich proximal erstreckende Widerhaken aufweist, die an der Innenfläche des Spritzenzylinders angreifen, und wobei das Verriegelungselement und der Stopfen derart angeordnet sind, dass die Kolbenstangenanordnung in bezug auf das Verriegelungselement proximal bewegbar ist.

4. Spritzenanordnung nach Anspruch 3, bei der das Verriegelungselement einen Körperteil mit einem distalen Ende und einem proximalen Ende aufweist, wobei der Körperteil des Verriegelungselements im wesentlichen V-förmig ist und in Angriff an jeder der beiden die Ausnehmung begrenzenden Flächen bringbar ist.

5. Spritzenanordnung nach Anspruch 4, mit einem ersten Paar von Schenkeln, die sich von dem proximalen Ende des Körperteils erstrecken und in bezug auf dieses auslenkbar sind, wobei die Schenkel an der Kolbenstangenanordnung angreifen.

6. Spritzenanordnung nach Anspruch 5, mit einem ersten sich proximal erstreckenden Widerhaken nahe dem distalen Ende des Körperteils und einem zweiten sich proximal erstreckenden Widerhaken, der sich von mindestens einem der Schenkel erstreckt, wobei der erste und der zweite Widerhaken an der Innenfläche des Spritzenzylinders angreifen.

7. Spritzenanordnung nach Anspruch 1, bei welcher der langgestreckte Körperteil der Kolbenstangeanordnung mehrere sich radial erstreckende Wände, die nahe der Längsachse konvergieren, und eine erste zusätzliche Wand aufweist, die sich von einer der radial verlaufenden Wände in eine nicht-radiale Richtung erstreckt, wobei die erste zusätzliche Wand eine der die Ausnehmung begrenzenden Flächen bildet.

8. Spritzenanordnung nach Anspruch 7, mit einer zweiten zusätzlichen Wand, die sich von einer der radial verlaufenden Wände aus erstreckt, wobei die zweite zusätzliche Wand eine der die Ausnehmung begrenzenden Flächen bildet.

9. Spritzenanordnung nach Anspruch 8, bei der die zweite zusätzliche Wand im wesentlichen parallel zu der ersten zusätzlichen Wand verläuft.

10. Spritzenanordnung nach Anspruch 1, bei welcher der langgestreckte Körperteil der Kolbenstangenanordnung eine erste Wand proximal des Stopfens und mehrere zweite Wände aufweist, die von einer ersten Seite der ersten Wand abstehen, wobei zwei der zweiten Wände die Flächen der Ausnehmung bilden.

11. Spritzenanordnung nach Anspruch 10, bei der mindestens eine der Flächen der Ausnehmung mehrere Anschläge zum Angreifen an dem Verriegelungselement aufweist.

12. Spritzenanordnung nach Anspruch 10, bei der sich die zweiten Wände im wesentlichen senkrecht in bezug auf die erste Wand erstrecken.

13. Spritzenanordnung nach Anspruch 10, bei der sich die zweiten Wände von zwei Seiten der ersten Wand erstrecken und mehrere Ausnehmungen bilden, wobei jede der Ausnehmungen in der Lage ist, das Verriegelungselement aufzunehmen.

14. Spritzenanordnung nach Anspruch 10, bei der das Verriegelungselement einen im wesentlichen V-förmigen Körperteil aufweist, der in Angriff an jeder der beiden die Ausnehmung begrenzenden Flächen bringbar ist.

15. Spritzenanordnung nach Anspruch 12, bei der das Verriegelungselement einen im wesentlichen V-förmigen Körperteil aufweist, der in Angriff an jeder der beiden die Ausnehmung begrenzenden Flächen bringbar ist.

## Revendications

1. Ensemble formant seringue comprenant :
un cylindre de seringue présentant une surface intérieure définissant une chambre, une extrémité ouverte, et une extrémité distale ;
un ensemble formant tige de piston s'étendant à l'intérieur dudit cylindre de seringue , et comprenant une partie de corps allongée présentant un axe longitudinal, une butée reliée à ladite partie de corps allongée, et un évidement s'étendant axialement, ledit évidement étant délimité par une paire de surfaces et étant radialement à l'écart de l'axe longitudinal de ladite partie de corps allongée, et
un élément bloquant positionné de manière coulissante à l'intérieur de ladite chambre, et s'étendant à l'intérieur dudit évidement, ledit élément bloquant étant en prise avec ladite surface intérieure dudit cylindre de seringue, de telle sorte que ledit élément bloquant est sensiblement immobile en direction de l'extrémité ouverte dudit cylindre de seringue, ledit élément bloquant pouvant en outre être mis en prise avec au moins une desdites surfaces dudit ensemble formant tige de piston, de telle sorte que lesdits ensemble formant tige de piston et élément bloquant peuvent être déplacés ensemble distalement vers l'extrémité distale dudit cylindre de seringue, ledit élément bloquant étant maintenu dans ledit évidement et sensiblement déplacé à l'écart de l'axe longitudinal dudit ensemble formant tige de piston.

2. Ensemble formant seringue selon la revendication 1, dans lequel ledit élément bloquant est composé d'une structure métallique résiliente monobloc, ledit élément bloquant étant positionné de telle sorte que ledit ensemble formant tige de piston peut être déplacé de manière proximale par rapport au dit élément bloquant.

3. Ensemble formant seringue selon la revendication 1, dans lequel ledit élément bloquant comprend un ou plusieurs ardillons s'étendant de manière proximale, en prise avec ladite surface intérieure dudit cylindre de seringue, et lesdits élément bloquant et butée sont positionnés de telle sorte que ledit ensemble formant tige de piston peut être déplacé de manière proximale par rapport au dit élément bloquant.

4. Ensemble formant seringue selon la revendication 3, dans lequel ledit élément bloquant comprend une partie de corps présentant une extrémité distale et une extrémité proximale, ladite partie de corps dudit élément bloquant étant généralement en forme de V et pouvant être mise en prise avec chacune de ladite paire de surfaces délimitant ledit évidement.

5. Ensemble formant seringue selon la revendication 4, comprenant une première paire de pattes s'étendant à partir de, et pouvant être défléchie par rapport à, ladite extrémité proximale de ladite partie de corps, lesdites pattes mettant en prise ledit ensemble formant tige de piston.

6. Ensemble formant seringue selon la revendication 5, comprenant un premier ardillon s'étendant de manière proximale, adjacent à ladite extrémité distale de ladite partie de corps, et un second ardillon s'étendant de manière proximale, à partir d'au moins une desdites pattes, lesdits premier et second ardillons étant en prise avec ladite surface intérieure dudit cylindre de seringue.

7. Ensemble formant seringue selon la revendication 1, dans lequel ladite partie de corps allongée dudit ensemble formant tige de piston comprend une pluralité de parois s'étendant radialement, qui convergent à proximité dudit axe longitudinal, et une première paroi supplémentaire s'étendant à partir d'une desdites parois s'étendant radialement, dans un sens non radial, ladite première paroi supplémentaire définissant une desdites surfaces délimitant ledit évidement.

8. Ensemble formant seringue selon la revendication 7, comprenant une seconde paroi supplémentaire s'étendant à partir d'une desdites parois s'étendant radialement, ladite seconde paroi supplémentaire définissant une desdites surfaces délimitant ledit évidement.

9. Ensemble formant seringue selon la revendication 8, dans lequel ladite seconde paroi supplémentaire est sensiblement parallèle à ladite première paroi supplémentaire.

10. Ensemble formant seringue selon la revendication 1, dans lequel ladite partie de corps allongée dudit ensemble formant tige de piston comprend une première paroi proximale par rapport à ladite butée, et une pluralité de secondes parois faisant saillie d'un premier côté de la première paroi, deux desdites secondes parois définissant lesdites surfaces dudit évidement.

11. Ensemble formant seringue selon la revendication 10, dans lequel au moins une desdites surfaces dudit évidement comprend une pluralité de butées destinées à être en prise avec ledit élément bloquant.

12. Ensemble formant seringue selon la revendication 10, dans lequel lesdites secondes parois s'étendent de manière sensiblement perpendiculaire par rapport à ladite première paroi.

13. Ensemble formant seringue selon la revendication 10, dans lequel lesdites secondes parois s'étendent à partir de deux côtés de ladite première paroi et définissent une pluralité d'évidements, chacun desdits évidements pouvant recevoir ledit élément bloquant.

14. Ensemble formant seringue selon la revendication 10, dans lequel ledit élément bloquant comprend une partie de corps généralement en forme de V pouvant se mettre en prise avec chacune de ladite paire de surfaces délimitant ledit évidement.

15. Ensemble formant seringue selon la revendication 12, dans lequel ledit élément bloquant comprend une partie de corps généralement en forme de V pouvant se mettre en prise avec chacune de ladite paire de surfaces délimitant ledit évidement.
